Europäisches Patentamt

European Patent Office

Office européen des brevets

(11) Veröffentlichungsnummer: **0 296 481**
**A2**

## (12) EUROPÄISCHE PATENTANMELDUNG

(21) Anmeldenummer: 88109599.6

(22) Anmeldetag: 16.06.88

(51) Int. Cl.⁴: **C12Q 1/00 , C12Q 1/32 , C12Q 1/28 , C12Q 1/26**

(30) Priorität: 20.06.87 DE 3720506

(43) Veröffentlichungstag der Anmeldung:
28.12.88 Patentblatt 88/52

(84) Benannte Vertragsstaaten:
**DE FR GB**

(71) Anmelder: **Drägerwerk Aktiengesellschaft
Moislinger Allee 53-55
D-2400 Lübeck 1(DE)**

(72) Erfinder: **Rindt, Klaus-Peter, Dr. rer. nat.
Dipl.-Bio.
Brömbsenstrasse 21
D-2400 Lübeck(DE)**

(54) Verfahren zum Nachweis gasförmiger Stoffe mittels einer enzymatischen Redox-Reaktion.

(57) Ein Verfahren zum Nachweis eines in wäßriger Lösung vorliegenden Stoffes, der mit einem auf einem Trägersubstrat aufgebrachten Enzym zu einer enzymatischen Redox-Reaktion mittels eines Elektronen-Akzeptor/Donator-Komplexes gebracht wird, der nach erfolgter Redox-Reaktion mit einem Chromogen zu einem Farbstoff reagiert, soll dahin verbessert werden, daß auch gasförmige Schadstoffe nachgewiesen werden können. Die Auswahl geeigneter Enzyme soll wegen deren "Schlüssel-Schloß-Codierung" ein empfindliches, gasartspezifisches Nachweisverfahren auch bei geringen Schadstoffkonzentrationen ermöglichen. Dies wird dadurch erreicht, daß

a) zunächst das Enzym auf dem Substratträger mit einer wäßrigen Reagenzlösung, welche den Komplex und das Chromogen enthält, und anschließend

b) die Luftprobe zur Überleitung des Schadstoffes aus der Gasphase in die Flüssigkeitsphase mit der Reagenzlösung in Verbindung gebracht wird.

## Verfahren zum Nachweis gasförmiger Stoffe mittels einer enzymatischen Redox-Reaktion

Die Erfindung betrifft ein Verfahren zum Nachweis eines in wäßriger Lösung vorliegenden Stoffes, der mit einem auf einem Trägersubstrat aufgebrachten Enzym zu einer enzymatischen Redox-Reaktion mittels eines Elektronen-Akzeptor/Donator-Komplexes gebracht wird, der nach erfolgter Redox-Reaktion zu einer Farbreaktion mit einem Chromogen führt. Eine Vorrichtung zur Durchführung des Verfahrens wird angegeben.

Derartige Verfahren werden beispielsweise zum Nachweis von Stoffwechselprodukten in Körperflüssigkeiten eingesetzt, wie in der EP-A 78 971 beschrieben ist.

Dort wird ein Testpapier mit einem Enzym, einem Chromogen und weiteren Zusätzen versehen, so daß für den Fall des Nachweises von Glukose das entsprechende Enzym Glukose-Oxidase zu einer Oxydation von Glukose führt, wodurch u. a. Wasserstoffperoxid entsteht, welches wiederum unter Anwesenheit einer Peroxidase ein Chromogen zu einem Farbstoff oxidiert.

Die bekannten Teststreifen sind lediglich für die Bestimmung von Stoffen einsetzbar, welche in flüssiger Phase vorliegen, so daß das für die enzymatische Umsetzung erforderliche Wasser durch die Probe selbst bereitgestellt wird. Der nachzuweisende Stoff liegt in relativ hoher Konzentration vor ($10^{-4}$ bis $10^{-1}$ M), so daß keine hohen Anforderungen an die Nachweisempfindlichkeit des enzymatischen Reaktionssystems gestellt zu werden brauchen.

Der vorliegenden Erfindung liegt somit die Aufgabe zugrunde, ein Verfahren zum Nachweis eines in wäßriger Lösung vorliegenden Stoffes derart zu verbessern, daß es für die Bestimmung eines Schadstoffes in gasförmiger Phase geeignet ist, um die stoffspezifischen enzymatischen Reaktionen für die Schadstoffmessung in Luft zugänglich zu machen. Die Auswahl geeigneter Enzyme soll wegen ihrer "Schlüssel-Schloß-Codierung" ein empfindliches, gasartspezifisches Nachweisverfahren auch bei geringen Schadstoffkonzentrationen ermöglichen.

Die Lösung der Aufgabe erfolgt mit den kennzeichnenden Verfahrensschritten des Hauptanspruches.

Der Vorteil der Erfindung liegt im wesentlichen darin, daß ein hochempfindliches, gasartspezifisches Nachweisverfahren auf der Basis einer enzymatischen Redox-Reaktion geschaffen wurde, welches sich durch fehlende Querempfindlichkeit zu anderen, in der zu untersuchenden Luftprobe befindlichen Gasarten auszeichnet. Durch Wahl eines geeigneten Enzyms für ein nachzuweisendes Gas "paßt" der "Schlüssel" des entsprechenden Gases nur in das für dieses geeignete "Schloß" des dazugehörigen Enzyms. Ein anderes Gas, mag es in noch so hohen Konzentrationen vorliegen, paßt nicht zu diesem Enzym und wird deswegen auch nicht an der Redox-Reaktion teilnehmen. Die Verfärbung des Chromogens kann daher ausschließlich einem einzigen Gas zugeordnet werden.

Die Bestimmung eines Schadstoffes aus einer Luftprobe kann so ablaufen, daß die wäßrige Reagenzlösung mit dem Enzym, dem Elektronen-Akzeptor-Donator-Komplex und dem Chromogen zunächst separat angesetzt und das Trägersubstrat mit dieser Lösung versehen wird. Alternativ kann auch das Enzym von vornherein an dem Trägersubstrat adsorptiv oder kovalent gebunden sein, beispielsweise durch Gefriertrocknung des Enzyms auf dem Trägersubstrat, so daß in diesem Falle die wäßrige Lösung nur noch den Akzeptor-Donator-Komplex und das Chromogen zu enthalten braucht. Bei der anschließenden Überleitung der Luftprobe über die Reagenzlösung tritt der gasförmige Schadstoff in die Flüssigkeitsphase ein und läßt nun die enzymatische Redox-Reaktion und die anschließende Farbreaktion des Chromogens ablaufen.

Als Trägersubstrat können alle Materialien eingesetzt werden, die in der Lage sind, das Enzym adsorptiv oder kovalent zu binden. Hierfür haben sich besonders Trägermaterialien aus Acrylharz in Form von Perlen u. a. wegen der guten Bindungskapazitäten als geeignet erwiesen. Ein solches Acrylharz als Trägersubstrat kann sämtliche Bestandteile der Reagenzlösung in lyophilierter Form gebunden haben, so daß zur Herstellung der wäßrigen Reagenzlösung der Acrylharzträger lediglich mit Wasser benetzt zu werden braucht. In der Regel ist der Reagenzlösung noch ein Puffer zuzusetzen, um einen für den Ablauf der enzymatischen Reaktion günstigen Bereich für den pH-Wert zwischen etwa 5 und 9 aufrechtzuerhalten. Hierzu sind Phosphatpuffer, Glyzin, Tris und GOOD-Puffer geeignet (vgl. hierzu: Methods in Enzymology, 104 (1984), 404-414).

Zur Durchführung des Verfahrens sind verschiedene Vorrichtungen geeignet, von denen eine besonders vorteilhafte Ausführungsform später erläutert wird. Für den Fall, daß dazu ein mit dem Trägersubstrat und der Reagenzlösung gefülltes Röhrchen benutzt wird, durch welches die Luftprobe gedrückt oder gesaugt wird, kann es während des Meßvorganges zum Austreiben mindestens eines Teiles der Reagenzlösung kommen, so daß die einmal gestartete enzymatische Reaktion wegen der dann abnehmenden Menge an Reagenzbestandteilen zum Erliegen kommt, so daß der gewünschte Farbumschlag des Chromogens nicht vollständig ist. Um diesen Nachteil zu beheben, ist es zweckmäßig, nach erfolgtem Transport der Luftprobe durch den Substratträger nochmals eine Entwicklerlösung auf den Substratträger zu bringen, welche zumindest den Akzeptor-Donator-Komplex und das Chromogen enthält. Die vorher unterbrochene enzymatische Reaktion wird dann weiter fortgesetzt, so daß es unter vollständiger Umsetzung der Reaktionsprodukte und zu einer vollständigen Farbreaktion kommt.

Zum Nachweis von Kohlenstoffmonoxid sind in der Reagenzlösung als Enzym die CO-Dehydrogenase, als Elektronen-Akzeptor-Komplex das (Methoxy)-Phenazinmethosulfat und als Chromogen ein Tetrazoliumsalz vorhanden. In diesem Fall wird CO unter Anwesenheit der Dehydrogenase zu $CO_2$ oxidiert, wobei von dem Akzeptor-Komplex ein Elektron aufgenommen wird. Der so reduzierte Komplex gibt sein Elektron an das Tetrazoliumsalz ab, wird also wieder oxidiert, wobei das Tetrazoliumsalz zu einem gefärbten Formazan umgebildet wird. Der synthetische Akzeptor-Komplex dient also als Katalysator zwischen dem Enzym und dem Chromogen.

Für den Nachweis von Formaldehyd wird die Formaldehyd-Dehydrogenase als Enzym benutzt, die jedoch als Akzeptor-Komplex zusätzlich zum Phenazinmethosulfat als synthetischen Akzeptor zusätzlich Nicotinamid-adenin-dinucleotid (NAD) als biologischen Akzeptor benötigt. Damit ist sichergestellt, daß durch die "Schlüssel-Schloß-Codierung" des Enzyms zwischen dem Formaldehyd und den Akzeptoren-Komplexen ein gasartspezifischer Nachweis für Formaldehyd ermöglicht wird. Formaldehyd wird durch das Enzym Formaldehyd-Dehydrogenase zu Ameisensäure oxidiert, gibt dabei an den NAD-Akzeptor ein Elektron ab, wodurch $NADH_2$ gebildet wird, welches seinerseits ein Elektron an den synthetischen Akzeptor abgibt und somit reduziert wird. Der reduzierte synthetische Akzeptor gibt ein Elektron an das Tetrazoliumsalz ab, wird dabei oxidiert und gefärbtes Formazan zeigt die Gegenwart von Formaldehyd an. Beide Akzeptoren werden nach erfolgter Nachweisreaktion in ihren ursprünglichen Zustand wieder zurückgeführt, so daß sie als Katalysatoren für die Enzymreaktion wirken.

In weiterer Ausgestaltung der Erfindung wird zum Nachweis von Wasserstoffperoxid in der Reagenzlösung das Enzym Peroxidase gegeben, wobei zusätzlich Aminoantipyrin zugefügt wird, welches gleichzeitig als Elektronen-Donator-Komplex und als Chromogen wirkt.

Die Enzym-Reaktion von $H_2O_2$ zu $H_2O$ kann mit solchen Enzymreaktionen gekoppelt werden, welche als Endprodukt u. a. auch $H_2O_2$ liefern. So kann vorteilhafterweise der Nachweis von Schwefeldioxid dadurch erfolgen, daß in der Reagenzlösung als Enzym sowohl die Sulfitoxidase als auch die Peroxidase und das Aminoantipyrin gleichzeitig als Donator-Komplex und Chromogen sowie ein Kupplungsreagenz in Form eines Phenol-, Naphtoloder Toluidinderivats, z. B. N-Ethyl-N-sulfopropyl-m-toluidin (vgl. US-4 587 100), vorliegen. In einer ersten Enzymreaktion wird aus dem Schwefeldioxid mit dem vorliegenden Wasser Sulfit gebildet, welches durch die Sulfitoxidase zu Sulfat umgewandelt wird, wobei Elektronen auf $O_2$ übertragen werden und $H_2O_2$ entsteht. Dieses $H_2O_2$ bildet mit Hilfe der Peroxidase Wasser, wobei das Aminoantipyrin ein Elektron abgibt, somit oxidiert wird und mit dem Kupplungsreagenz zu einer verfärbten Verbindung kuppelt.

Eine Vorrichtung zur Durchführung des Nachweisverfahrens kann zweckmäßigerweise, in Anlehnung an die Ausführungsform der bekannten kolorimetrischen Prüfröhrchen, aus einem röhrchenförmigen Behälter bestehen, der an zwei Enden geöffnet werden kann. Dieser Behälter ist mit dem Substratträger gefüllt, beispielsweise in Form einer Schüttung aus porösen Acrylharzkugeln. Die Schüttung ist mit der Reagenzlösung getränkt, so daß in ihr die notwendigen Bestandteile, wie das Enzym, der Elektronen-Donator/Akzeptor-Komplex, das Chromogen, und eine geeignete Pufferlösung enthalten sind. Die Reagenzlösung wird nach dem einseitigen Öffnen des vorher geschlossenen Behälters auf den darin befindlichen Substratträger gegeben. Zum Nachweis des zu untersuchenden Schadstoffes wird der Behälter an seinem zweiten zu öffnenden Ende an eine Fördereinrichtung, beispielsweise eine Pumpe, angeschlossen und die Luftprobe durch den Behälter hindurchgesaugt oder gedrückt. Während der Förderung der Luftprobe durch den Substratträger geht der gasförmige Schadstoff in Lösung und führt zu einer Farbreaktion in der Reagenzlösung.

Während dieses Meßvorganges kann es auftreten, daß ein Teil der Reagenzlösung mit ausgetrieben wird, so daß die Farbreaktion nicht vollständig ablaufen kann, weil die notwendigen an der Reaktion beteiligten Substanzen nicht mehr im stöchiometrischen Verhältnis vorliegen.

So kann es z. B. zu einer Verarmung an Wasser kommen, was zu einer Einschränkung der Diffusion führt. Die Färbung kann sich nur partiell ausbilden, obwohl das enzymatisch gebildete Primärprodukt entstanden ist. Diesem Mangel kann abgeholfen werden, indem in den Behälter nochmals eine Menge an Nachweisreagenz als Entwicklerlösung eingegeben wird.

Während bei den bekannten Nachweisvorrichtungen in Form von Teststreifen der nachzuweisende Stoff in der Flüssigkeit gelöst und in für die enzymatische Reaktion genügend hoher Konzentration vorliegt, ist hingegen bei der Untersuchung von gasförmigen Schadstoffen mit erheblich niedrigeren Konzentrationswerten zu rechnen. Um auch solche geringen Schadstoffmengen nachweisen zu können, ist es zweckmäßig, sie vorher in einem Sammelbehälter zu sammeln. Dieser Sammler besteht vorteilhafterweise aus einem Röhrchen, welches mit einem Molekularsieb gefüllt ist und durch den eine genügend große Luftprobe gezogen wird, um die entsprechende Menge an Schadstoffen zu sammeln, so daß bei der nachfolgenden enzymatischen Reaktion eine unterscheidbare Verfärbung auftreten kann. Nachdem die Luftprobe durch den Sammler geleitet wurde und die Schadstoffe in ihm adsorbiert wurden, kann der Sammler mit dem röhrchenförmigen Behälter in Verbindung gebracht werden. Der adsorbierte Schadstoff wird mit destilliertem Wasser ausgewaschen oder auf sonst geeignete Weise ausgetrieben und zusammen mit der Reagenzlösung in den Prüfbehälter gebracht.

Ein Ausführungsbeispiel der Erfindung wird anhand der schematischen Zeichnung dargestellt und im folgenden näher erläutert.

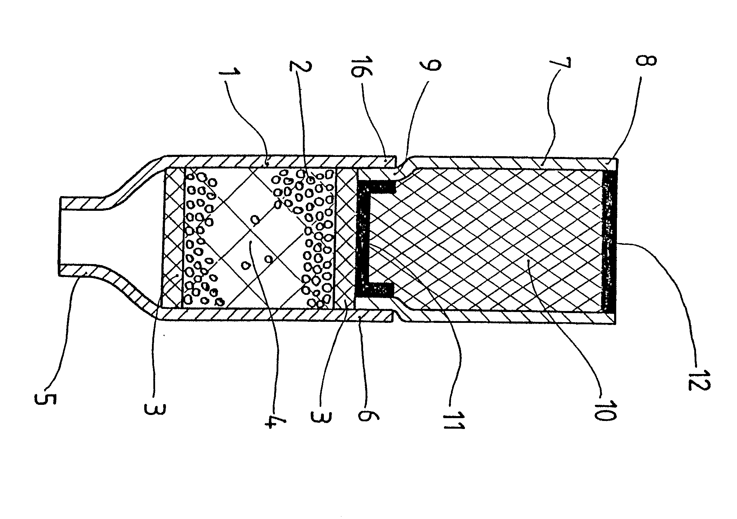

Die einzige Figur zeigt im Schnitt den Prüfbehälter (1) in Form eines durchsichtigen Röhrchens, welcher mit einer Trägersubstanz (2), dargestellt in Form von kleinen Kügelchen aus Acrylharz, gefüllt ist. Die Trägersubstanz (2) ist zwischen zwei porösen Halteelementen (3) eingebettet, und mit einer Reagenzlösung (4) getränkt. Der Prüfbehälter (1) besitzt zwei öffenbare Enden (5, 6), von denen das eine (6) eine Schulter (16) trägt, in welcher ein Sammler (7) einsetzbar ist. Der Sammler (7) ist ebenfalls an seinen beiden Enden (8, 9) öffenbar und mit einem Molekularsieb (10) gefüllt, welches zwischen zwei porösen Stopfen (11, 12) gehalten ist.

Nachfolgend werden drei Beispiele für die Messung von verschiedenen Gasen, Formaldehyd, Kohlenstoffmonoxid und Wasserstoffperoxid angegeben:

Beispiel 1

Formaldehydmessung

20 Einheiten Formaldehyddehydrogenase (gereinigt aus Pseudomonas putida C-83 oder Arthrobacter spec.) werden an 10 g vernetztes Polyacrylamid, gequollen in Phosphatpuffer (0.05 Mol x $L^{-1}$ pH 7.2) und in Gegenwart von 0.5 Mol x $L^{-1}$ KCl gebunden. Das gewaschene und abgesaugte Material wird in das Prüfröhrchen (1) bis zu einer Höhe von 2-3 cm gefüllt und mit der porösen Abdeckung (3) versehen. Vor der Begasung wird das Röhrchen (1) mit 2 mL einer Lösung der folgenden Zusammensetzung äquilibriert: Phosphatpuffer (0.05 Mol x $L^{-1}$ pH 7.2), NAD (2 mMol x $L^{-1}$), 1-Methoxyphenazin-methosulfat, MO-PMS (0.05 mMol x $L^{-1}$), p-Jodnitrotetrazoliumchlorid, INT (0.2 mMol x $L^{-1}$). Nach Durchsaugen einer definierten Prüfgas- oder Probegasmenge (0.1-10 L) mit einer Geschwindigkeit von 0.05 - 0.2 L x $min^{-1}$ ergibt sich eine Färbung, die in ihrer Intensität der Menge an Formaldehyd in der Probe proportional ist. Speziell bei geringen Konzentrationen (entsprechend großen Probevolumina, die zu stärkerem Wasserverlust führen) ist eine Nachentwicklung mit 0.5 - 1 mL der oben genannten Lösung erforderlich.

Beispiel 2

CO-Messung

20 Einheiten CO-Dehydrogenase, gereinigt nach bekannten Verfahren z. B. aus Pseudomonas carboxidoflava, werden wie in Beispiel 1 beschrieben, an das Trägermaterial (2) gebunden und in das Röhrchen (1) gefüllt. Die Äquilibrierung erfolgt mit der gleichen Lösung wie in Beispiel 1, nur enthält diese kein NAD. Nach entsprechender Begasung mit CO-haltigem Gas tritt eine Färbung wie in Beispiel 1 auf. Die Umsetzung mit CO ist unter diesen Umständen nicht quantitativ, aber ein Nachweis im Bereich des MAK-Wertes (30 ppm) ist bereits mit 0.1-0.2 L Probevolumen möglich. Nachentwicklung wie in Beispiel 1, falls erforderlich.

Beispiel 3

$H_2O_2$-Messung

10 mg Peroxidase aus Meerrettich (Sigma Typ II) werden, wie in Beispiel 1 beschrieben, an den Träger (2) gebunden und in das Röhrchen (1) gefüllt. Sie werden mit 1-2 mL einer Lösung folgender Zusammensetzung äquilibriert: Phosphatpuffer (0.1 mMol x $L^{-1}$ pH 7.2), Na-Citrat 0.1 mMol x $ML^{-1}$), Aminoantipyrin (0.4 mMol x $L^{-1}$), N-Ethyl-N-Sulfopropyl-m-toluidin, TOPS (0.4 mMol x $L^{-1}$). Nach Begasung mit $H_2O_2$-haltigem Gas und ggf. Nachentwicklung mit oben genannter Lösung ergibt sich eine Färbung, die in ihrer Intensität der $H_2O_2$-Konzentration proportional ist.

Ansprüche

1. Verfahren zum Nachweis eines in wäßriger Lösung vorliegenden Stoffes, der mit einem auf einem Trägersubstrat aufgebrachten Enzym zu einer enzymatischen Redox-Reaktion mittels eines Elektronen-Akzeptor/Donator-Komplexes gebracht wird, der nach erfolgter Redox-Reaktion mit einem Chromogen zu einem Farbstoff reagiert, dadurch gekennzeichnet, daß zur Bestimmung eines Schadstoffes aus einer Luftprobe zunächst

a) das auf dem Substratträger befindliche Enzym mit einer wäßrigen Reagenzlösung, enthaltend den Elektronen-Akzeptor/Donator-Komplex und das Chromogen, und anschließend

b) die Luftprobe zur Überleitung des Schadstoffes aus der Gasphase in die Flüssigkeitsphase mit der Reagenzlösung in Verbindung gebracht wird.

2. Verfahren nach Anspruch 1, dadurch gekennzeichnet, daß nach erfolgten Verfahrensschritten a) und b)
der Substratträger nochmals

c) mit einer Entwicklerlösung, die zumindest den Elektronen-Akzeptor/Donator-Komplex und das Chromogen enthält, versehen wird.

3. Verfahren nach Anspruch 1 oder 2, dadurch gekennzeichnet, daß zum Nachweis von Kohlenstoffmonoxid in der Reagenzlösung als Enzym die CO-Dehydrogenase, als Elektronen-Akzeptor-Komplex das (Methoxy)-Phenazinmethosulfat und als Chromogen ein Tetrazoliumsalz vorliegen.

4. Verfahren nach Anspruch 1 oder 2, dadurch gekennzeichnet, daß zum Nachweis von Formaldehyd in der Reagenzlösung als Enzym die Formaldehyd-Dehydrogenase, als Elektronen-Akzeptor-Komplex sowohl Nicotinamid-adenindinucleotid (NAD) als auch (Methoxy)-Phenazinmethosulfat, und als Chromogen ein Tetrazoliumsalz vorliegen.

5. Verfahren nach Anspruch 1 oder 2, dadurch gekennzeichnet, daß zum Nachweis von $H_2O_2$ in der Reagenzlösung als Enzym die Peroxidase, als Elektronen-Donator-Komplex und als Chromogen Aminoantipyrin sowie als Kupplungsreagenz ein Phenol-, Naphtol- oder ein Toluidinderivat vorliegen.

6. Verfahren nach Anspruch 1 oder 2, dadurch gekennzeichnet, daß zum Nachweis von Schwefeldioxid in der Reagenzlösung als Enzym sowohl die Sulfitoxidase als auch die Peroxidase, als Elektronen-Donator-Komplex und als Chromogen Aminoantipyrin sowie als Kupplungsreagenz ein Phenol-, Naphtol- oder ein Toluidinderivat vorliegen.

7. Verfahren nach einem der Ansprüche 1 bis 6, dadurch gekennzeichnet, daß der Schadstoff aus der Luftprobe in einem Sammler gesammelt und zur Durchführung der Verfahrensschritte a) bis c) aus dem Sammler ausgetrieben wird.

8. Vorrichtung zur Durchführung des Verfahrens nach Anspruch 1, dadurch gekennzeichnet, daß in einem zweiseitig öffenbaren röhrchenförmigen und durchsichtigen Prüfbehälter (1) ein poröser, mit einem Enzym belegter Substratträger (2) gefüllt ist, der mit einer wäßrigen Reagenzlösung (4), enthaltend das Enzym, den Elektronen-Donator-Akzeptor-Komplex und das Chromogen, versehen ist, und durch den zum Nachweis von gasförmigen Schadstoffen eine Luftprobe förderbar ist.

9. Vorrichtung nach Anspruch 8, dadurch gekennzeichnet, daß an dem Eintrittsende (6) des Behälters (1) ein mit dem gasförmigen Schadstoff beladener Sammler (7) vorgesehen ist.